# EUROPEAN PATENT APPLICATION

(11) **EP 1 862 801 A1**
(43) Date of publication of application: **05.12.2007**
(21) Application number: 06715198.5
(22) Date of filing: 03.03.2006
(51) Int. Cl.: G01N 33/542, C12N 5/10, C12N 15/09, C12Q 1/02, G01N 21/78, G01N 33/53

(54) **REPORTER GENE ASSAY METHOD**

(30) Priority: 07.03.2005 JP 2005062062
(71) Applicant: Shionogi Co., Ltd., Osaka-shi, Osaka 541-0045 (JP)
(72) Inventor: ENOMOTO, Koji, c/o Shionogi & Co.,Ltd., Osaka-shi, Osaka 5530002 (JP); NUMATA, Yoshito, c/o Shionogi & Co.,Ltd., Osaka-shi, Osaka, 5530002 (JP); TAKEMOTO, Hiroshi, c/o Shionogi & Co.,Ltd., Osaka-shi, Osaka, 5530002 (JP)
(74) Representative: Vossius & Partner
(86) International application number: PCT/JP2006/304115
(87) International publication number: WO 2006/095654

(57) **Abstract**

The invention provides a more highly accurate assay for measuring the transcriptional activity of a test substance. Disclosed is a reporter gene assay, comprising the steps of contacting a cell having a vector wherein a reporter gene containing a gene encoding an epitope tag is ligated downstream to a recognition sequence of a transcription factor and a nucleotide sequence necessary for transcriptional initiation, with a test substance and detection antibodies; detecting a phenomenon caused by the two kinds of detection antibodies coming close to each other; and correlating the detected phenomenon with the effect of the test substance on transcriptional regulatory mechanism.

SEQUENCE LISTING

<110>  Shionogi&Co., Ltd.

<120>  Reporter gene assay

<130>  N2704 EP S3

<160>  17

<170>  PatentIn version 3.1

<210>  1
<211>  9
<212>  PRT
<213>  Artificial

<220>
<223>  polypeptide for antigen

<400>  1

Gly Glu Pro Gly Asp Asp Ala Pro Ser
1               5


<210>  2
<211>  7
<212>  PRT
<213>  Artificial

<220>
<223>  polypeptide for antigen

<220>
<221>  MOD_RES
<222>  (3)..(3)
<223>  4Hyp


<400>  2

Gly Pro Pro Gly Pro Gln Gly
1               5


<210>  3
<211>  19
<212>  PRT
<213>  Artificial

<220>
<223>  polypeptide for epitope tag

<400>  3

Gly Glu Pro Gly Asp Asp Gly Pro Ser Gly Ala Glu Gly Pro Pro Gly
1               5                   10                  15

Pro Gln Gly

<210>  4
<211>  116
<212>  PRT
<213>  Mus musculus

<400>  4

Glu Val Gln Leu Val Glu Ser Gly Gly Ser Leu Val Gln Pro Gly Gly
1               5                   10                  15

Ser Leu Lys Leu Ser Cys Ala Ala Ser Gly Phe Thr Phe Asn Asn Tyr
            20                  25                  30

Gly Met Ser Trp Val Arg Gln Ile Pro Asp Lys Arg Leu Glu Leu Val
        35                  40                  45

Ala Thr Ile Thr Gly Asn Gly His Ser Thr Tyr Tyr Pro Val Ser Val
    50                  55                  60

Arg Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Val Arg Gly Tyr Ser Asn Phe Ala Phe Trp Gly Gln Gly Thr Thr Leu
            100                 105                 110

Thr Val Ser Ser
        115

<210>  5
<211>  113
<212>  PRT
<213>  Mus musculus

<400>  5

Asp Val Val Leu Thr Gln Thr Pro Leu Thr Leu Ser Leu Thr Ile Gly
1               5                   10                  15

Gln Pro Ala Ser Ile Ser Cys Lys Ser Ser Gln Ser Leu Leu Asn Ser

            20                  25                  30

Asp Gly Glu Thr Tyr Leu Asn Trp Leu Leu Gln Arg Pro Gly Gln Ser
        35                  40                  45

Pro Lys Arg Leu Ile Tyr Leu Val Ser Lys Leu Gly Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Cys Gln Gly
                85                  90                  95

Thr His Phe Pro Trp Thr Phe Gly Gly Gly Thr Lys Leu Glu Ile Lys
            100                 105                 110

Arg

<210> 6
<211> 120
<212> PRT
<213> Mus musculus

<400> 6

Gln Val Thr Leu Lys Glu Ser Gly Pro Gly Ile Leu Gln Pro Ser Gln
1               5                   10                  15

Thr Leu Ser Leu Thr Cys Ser Phe Ser Gly Phe Ser Leu Ser Thr Tyr
            20                  25                  30

Gly Met Gly Val Gly Trp Ile Arg Gln Pro Ser Gly Lys Gly Leu Glu
        35                  40                  45

Trp Leu Ala His Ile Trp Trp Asn Asp Asn Lys Tyr Tyr Asn Thr Thr
    50                  55                  60

Leu Lys Ser Arg Leu Thr Ile Ser Lys Asp Thr Ser Asn Lys Gln Val
65                  70                  75                  80

Phe Leu Lys Ile Ala Ser Val Asp Thr Ala Asp Thr Ala Thr Tyr Tyr
                85                  90                  95

Cys Ala Arg Ile Glu Arg Trp Asp Pro Trp Phe Ala Tyr Trp Gly Gln
            100                 105                 110

Gly Thr Leu Val Thr Val Ser Ala
        115                 120


<210>  7
<211>  113
<212>  PRT
<213>  Mus musculus

<400>  7

Asp Val Val Met Thr Gln Thr Pro Leu Ser Leu Pro Val Ser Leu Gly
1               5                   10                  15


Asp Gln Ala Ser Ile Ser Cys Arg Ser Ser Gln Ser Leu Val Tyr Ser
            20                  25                  30


Asn Gly Asn Thr Tyr Phe His Trp Tyr Leu Gln Lys Pro Gly Gln Ser
        35                  40                  45


Pro Lys Leu Leu Ile Tyr Lys Val Ser Asn Arg Phe Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Leu Gly Val Tyr Phe Cys Ser Gln Ser
                85                  90                  95


Thr His Val Pro Leu Thr Phe Gly Ala Gly Thr Lys Leu Glu Leu Lys
            100                 105                 110


Arg



<210>  8
<211>  24
<212>  DNA
<213>  Artificial

<220>
<223>  primer for cDNA synthesis

<400>  8
cagggtcacc atggagttag tttg                                              24


<210>  9
<211>  24
<212>  DNA

<213>  Artificial

<220>
<223>  primer for cDNA synthesis

<400>  9
tccagagttc caagtcacag tcac                                              24


<210>  10
<211>  24
<212>  DNA
<213>  Artificial

<220>
<223>  primer for cDNA synthesis

<400>  10
gactgaggca cctccagatg ttaa                                              24


<210>  11
<211>  30
<212>  DNA
<213>  Artificial

<220>
<223>  primer for PCR

<400>  11
aggggccagt ggatagacag atgggggtgt                                        30


<210>  12
<211>  30
<212>  DNA
<213>  Artificial

<220>
<223>  primer for PCR

<400>  12
aggggccagt ggatagactg atgggggtgt                                        30


<210>  13
<211>  33
<212>  DNA
<213>  Artificial

<220>
<223>  primer for PCR

<400>  13
ggatggtggg aagatggata cagttggtgc agc                                    33


<210>  14
<211>  57
<212>  DNA

<213>  Artificial

<220>
<223>  Designed oligonucleotide to synthesize  epitope tag

<400>  14
ggagagcctg gagatgacgg tccctctggt gccgaaggtc caccaggtcc ccagggt          57


<210>  15
<211>  966
<212>  DNA
<213>  Artificial

<220>
<223>  DNA seaquence for reporter gene

<400>  15
atggagcccg gccccgacgg ccccgccgcc tccggccccg ccgccatccg cgagggctgg      60

ttccgcgaga cctgcagcct gtggcccggc caggccctgt cgctgcaggt ggagcagctg     120

ctccaccacc ggcgctcgcg ctaccaggac atcctcgtct tccgcagtaa gacctatggc     180

aacgtgctgg tgttggacgg tgtcatccag tgcacggaga gagacgagtt ctcctaccag     240

gagatgatcg ccaacctgcc tctctgcagc caccccaacc cgcgaaaggt gctgatcatc     300

gggggcggag atggaggtgt cctgcgggag gtggtgaagc acccctccgt ggagtccgtg     360

gtccagtgtg agatcgacga ggatgtcatc caagtctcca agaagttcct gccaggcatg     420

gccattggct actctagctc gaagctgacc ctacatgtgg gtgacggttt tgagttcatg     480

aaacagaatc aggatgcctt cgacgtgatc atcactgact cctcagaccc catgggcccc     540

gccgaaagtc tcttcaagga gtcctattac cagctcatga agacagccct caaggaagat     600

ggtgtcctct gctgccaggg cgagtgccag tggctgcacc tggacctcat caaggagatg     660

cggcagttct gccagtccct gttccccgtg gtggcctatg cctactgcac catccccacc     720

taccccagcg gccagatcgg cttcatgctg tgcagcaaga acccgagcac gaacttccag     780

gagccggtgc agccgctgac acagcagcag gtggcgcaga tgcagctgaa gtactacaac     840

tccgacgtgc accgcgccgc ctttgtgctg cccgagtttg cccgcaaggc cctgaatgat     900

gtgagcggag agcctggaga tgacggtccc tctggtgccg aaggtccacc aggtccccag     960

ggttga                                                                966


<210>  16
<211>  37
<212>  DNA
<213>  Artificial

<220>
<223>  IL-4 regulatory element

<220>
<221> protein_bind
<222> (11)..(19)
<223> CCAAT-enhancer binding site

<220>
<221> protein_bind
<222> (24)..(30)
<223> STAT6 binding site

<400> 16
gatcccgctg ttgctcaatc gacttcccaa gaacaga 37

<210> 17
<211> 157
<212> DNA
<213> Artificial

<220>
<223> timidine kinase promoter

<400> 17
agatcttcta tgatgacaca aaccccgccc agcgtcttgt cattggcgaa ttcgaacacg 60

cagatgcagt cggggcggcg cggtcccagg tccacttcgc atattaaggt gacgcgtgtg 120

gcctcgaaca ccgagcgacc ctgcagcgac ccgctta 157

## Description

### Technical Field

The present invention relates to a reporter gene assay.

### Background

When a cytokine, hormone, antigen or the like is bound to a receptor on a cell membrane, a reaction cascade in which second messengers such as calcium ion, cyclic AMP etc. and protein kinase are intricately intertwined with one another in a cell is activated to transmit information. At this time, a transcription factor located downstream of a signal transduction pathway receives the information, moves into a nucleus, and binds to a recognition sequence present in a specific translation initiation site, to promote transcription of a target gene. Such transcriptional regulatory mechanism plays an important role in reproduction, cell differentiation, energy metabolism, growth, and maintenance of biological homeostasis. Therefore, it is known that when transcriptional regulation is not normally performed, the expression level of the target gene is abnormalized thus resulting in various diseases and abnormalities. For developing therapeutic or prophylactic agents for such diseases etc, various assays for finding substances regulating transcription of the target gene are known in the art. Generally, such assay is called reporter gene assay, wherein a plasmid containing a DNA having a reporter gene ligated to regions (a promoter etc.) necessary for transcriptional initiation is introduced into a cell, and the amount of a reporter protein formed as a result of the transcription and translation of the reporter gene is used as an indicator to examine the action of a test substance. As the reporter gene, a gene for firefly luciferase, bacterial luciferase, green fluorescent protein (GFP), enhanced green fluorescent protein (EGFP), chloramphenicol acetyltransferase (CAT), alkaline phosphatase, β-glactosidase or the like is widely used (see "Handbook of Genetic Engineering", revised 4th edition, pp. 223-226, published by Yodosha Co., Ltd. (2003)).

In the conventional reporter gene assay, luminescence, fluorescence, or a change in the color of a reagent, accompanying the enzyme activity of a reporter protein, or a change in some phenotypes detectable in a cell into which the gene was introduced, was used as an indicator to determine the transcriptional activity. In these methods, however, the amount of the reporter protein formed is indirectly measured thus making accuracy problematic. For example, the ability of a test substance to regulate transcription may be sometimes erroneously evaluated due to unspecific signals caused by enzymes inherent in a cell or due to the influence of the test substance on the enzyme activity of a reporter gene. Accordingly, there is strong demand for an assay capable of solving such problems and measuring transcriptional activity easily and rapidly.

### Summary of Invention

The present inventors made extensive study, and as a result, they found that the above object can be achieved by the reporter gene assay described below, and the present invention was thereby completed.
That is, the present invention provides:
[1] a reporter gene assay, comprising the steps of:
   (a) contacting a cell having a vector wherein a reporter gene containing a gene encoding an epitope tag having a first epitope and a second epitopes is ligated downstream to a recognition sequence of a transcription factor and a nucleotide sequence necessary for transcriptional initiation, with a test substance, a detection antibody recognizing the first epitope and a detection antibody recognizing the second epitope;
   (b) detecting a phenomenon caused by both the detection antibodies binding to the first and second epitopes and coming close to each other; and
   (c) correlating the detected phenomenon with the effect of the test substance on transcriptional regulatory mechanism,
   wherein the first epitope and the second epitope are arranged such that upon binding of their recognizing detection antibodies thereto, both the detection antibodies can come close to each other;
[2] the method according to the above-mentioned [1], wherein the cell is allowed to express a member selected from the group consisting of a transcription factor, a ligand, a receptor and a coactivator.
[3] the method according to the above-mentioned [1] or [2], wherein both the detection antibodies are labeled with a phosphor.
[4] the method according to the above-mentioned [3], wherein the phosphor consists of a combination of a europium compound and an allophycocyanin derivative;
[5] a cell comprising a vector wherein a reporter gene containing a gene encoding an epitope tag having first and second epitopes is ligated downstream to a recognition sequence of a transcription factor and a nucleotide sequence necessary for transcriptional initiation;
[6] the cell according to the above-mentioned [5], wherein the reporter gene has a sequence set forth in SEQ ID NO: 15 in the Sequence Listing;
7. a vector for use in the method according to any of the above-mentioned [1] to [4]; and
8. a kit for use in the method according to any of the above-mentioned [1] to [4].

In the assay of the present invention, the amount of a reporter protein formed is measured directly, thus circumventing false recognition caused by various factors such as cells and test substances. In the assay of the present invention, the ability of a test substance to regulate transcription can be accurately measured by eliminating a measurement error (color quenching) attributable to the color of a test substance.

### Brief Description of Drawings

Fig. 1 is an illustration of a reporter plasmid.
Fig. 2 shows the results of measurement by the reporter gene assay of the present invention. Panel A is a graph showing the relationship between the time of stimulating cells with 20 ng/ml IL-4 and the ratio value. Panel B is a graph showing the relationship between the amount of IL-4 added for stimulating cells for 24 hours and the fluorescence intensity.

### Best Mode for Carrying Out the Invention

The present invention relates to a reporter gene assay, comprising the steps of: contacting a cell having a vector wherein a reporter gene containing a gene encoding an epitope tag having a first epitope and a second epitopes is ligated downstream to a recognition sequence of a transcription factor and a nucleotide sequence necessary for transcriptional initiation, with a test substance, a detection antibody recognizing the first epitope and a detection antibody recognizing the second epitope; detecting a phenomenon caused by both the detection antibodies binding to the first and second epitopes and coming close to each other; and correlating the detected phenomenon with the effect of the test substance on transcriptional regulatory mechanism, wherein the first epitope and the second epitope are arranged such that upon binding of their recognizing detection antibodies thereto, both the detection antibodies can come close to each other.

The above-mentioned method of the present invention can be practiced easily by utilizing a specific vector and kit, and the present invention also provides such vector and kit.

In the present invention, there is used a cell comprising (a) a vector wherein a reporter gene containing a gene encoding an epitope tag is ligated downstream to a recognition sequence of a transcription factor and a nucleotide sequence necessary for transcriptional initiation, wherein (b) the two epitopes are arranged such that upon binding of their recognizing detection antibodies to the epitope tag, both the detection antibodies can come close to each other.

The cell used in the method of the present invention comprises a vector (also referred to hereinafter as "reporter plasmid") having a reporter gene ligated downstream to a recognition sequence of a transcription factor and a nucleotide sequence for transcriptional initiation.

Such reporter plasmid can be prepared for example by introducing the above reporter gene into a region downstream of a DNA having a recognition sequence of a transcription factor and a DNA having a nucleotide sequence necessary for transcriptional initiation in a suitable vector.

The vector that can be used is a vector suitable for genetic engineering techniques with *Escherichia coli* etc. Specifically, there includes a commercial vector etc. having the origin of replication (replicon) capable of functioning in a microorganism and a drug resistance gene and having a gene insertion site (multi-cloning site) downstream thereof.

Alternatively, the reporter plasmid in the present invention can be constructed by inserting a gene encoding an epitope tag into a commercially available reporter plasmid.

A promoter and a transcriptional regulatory region (cis factor) are necessary for gene transcription. In the cis factor, there are an enhancer for activating transcription and a silencer for suppressing transcription. A factor (trans factor) binding to the cis factor is called a sequence-specific factor or a transcriptional regulatory factor, as opposed to a general transcription factor binding to a promoter. A large number of sequence-specific transcription factors (referred to hereinafter as transcription factor) have been reported, and have recognition sequences different from one another. The number of transcription factors that have been identified until now reaches several hundred. Known transcription factors include, for example, SRF, Sp1, HNF-1, STAT, NFκB etc. ("Igaku No Ayumi (Advance in Medicine)", vol. 190, No. 6 (1999), pp. 52-57). When such transcription factor or its complex recognizes and binds to a certain DNA sequence thereby promoting/suppressing the transcription of a target gene downstream thereof, the DNA sequence is referred to as "a recognition sequence of the transcription factor". In the assay of the present invention, such a sequence can be arbitrarily changed depending on the object. For attaining sufficient transcriptional performance, it is usually preferable that about 2 to 5 responsive elements of the transcription factor are ligated in tandem. A DNA having such nucleotide sequence can be prepared by chemical synthesis or by amplification with PCR and subsequent cloning, etc,

The "nucleotide sequence necessary for transcriptional initiation" refers to a nucleotide sequence which after a transcription factor binds to the above recognition sequence, is involved in the initiation or efficiency of transcription reaction of a target gene such as a reporter gene downstream thereof. This kind of nucleotide sequence is generally called a promoter or an enhancer. This kind of nucleotide sequence when involved in control of transcription of a downstream sequence should be ligated such that the downstream sequence can function. As regions having such properties, various sequences are known to those skilled in the art, and all of such sequences can be applied to the present invention. Examples of such sequences include not only a nucleotide sequence in the 5'-upstream region of a thymidine kinase gene (tk) in the Examples but also a nucleotide sequence of a 5'-upstream region of a glutathione S-transferase Ya subunit gene (Proc. Natl. Acad. Sci. USA, 87, 3826-3830 (1990)) and a nucleotide sequence in the 5'-upstream region of a cytochrome P4501A1 gene (Eur. J. Biochem., 159, 219-225 (1986)).

A region comprising the "recognition sequence of a transcription factor" and the "nucleotide sequence necessary for transcriptional initiation" wholly or partially integrated therein is also known, and in this case, this region can be applied as a whole to the method of the present invention. Examples of such region can include a cAMP response element (CRE), an estrogen receptor element (ERE), a serum response element (SRE) and a TPA response element (TRE). An IL-4 regulatory element used in the Examples below also contains a recognition sequence of STAT6 as a transcription factor and a binding sequence (enhancer) of CCAAT-enhancer-binding protein (C/EBP). A DNA having such nucleotide sequence can be prepared for example by designing and preparing, on the basis of a known nucleotide sequence, oligonucleotides for amplifying the DNA encoding an objective region and subsequently performing PCR with the prepared oligonucleotides as primers.

### Reporter Gene

The type of "reporter gene" used in the method of the present invention is not particularly limited, but it is important that a gene encoding an epitope tag be contained therein. The epitope tag is a polypeptide (tag) having a specific amino acid sequence which can be fused with a protein of interest, and its amino acid sequence contains a region (epitope) binding to an antibody.

The epitope tag used in the method of the present invention has at least two epitopes, wherein the respective epitopes are arranged such that upon binding of their recognizing detection antibodies thereto, both the detection antibodies can come close to each other. The terms "come close to each other" mean that a detection antibody capable of binding to a certain epitope (referred to hereinafter as "first epitope'') in a manner specific to immune reaction and a detection antibody capable of binding to another epitope (referred to hereinafter as "second epitope") in a manner specific to immune reaction are in such a positional relationship that both the detection antibodies can bind to their epitopes without steric hindrance and can detect the reaction occurring between their labels while the antibodies are bound to the epitopes.

In the present invention, a detection antibody recognizing the first epitope and a detection antibody recognizing the second epitope are used. The term "recognizing" means "immune-specific binding". The term "immune-specific binding" refers to the binding reaction between an antibody and an amino acid sequence, and this binding is an evidence of a target amino acid sequence in a mixed state such as a cell disrupted material. Accordingly, under specified conditions, the antibody binds predominantly to a specific sequence but does not bind in a significant amount to other amino acid sequences present in a sample. This interaction when used in reference to the reaction between an epitope and an antibody is referred to as immune-specific binding.

The two epitopes may be linked directly to each other or crosslinked with each other via a suitable peptide linker. The antibody generally has a molecular weight of about 150 kDa which is larger than that of an epitope tag so that because of the steric hindrance between the detection antibodies, the binding of the antibodies themselves to the epitopes may be hindered sometimes. Accordingly, a peptide linker having a suitable length is desirably inserted into between the first epitope and the second epitope. When the two epitopes are crosslinked with each other via a peptide linker, usually 0 to 20, preferably 3 to 6, amino acid residues are desirably inserted into between the epitopes. Although the amino acid residues usable as a linker are not particularly limited, those without crossreactivity with the detection antibodies and with a relative short side chain are selected. The amino acid sequences of the first epitope and second epitope are not limited to those derived from the same species and may be those derived from different species.

The reporter gene in the cell used in the method of the present invention can include, for example, a gene encoding a fusion protein having an arbitrary protein fused with the epitope tag. The arbitrary protein can be prepared by PCR wherein oligonucleotides for amplifying a gene encoding the protein, which are designed and prepared on the basis of a nucleotide sequence known in database etc., are used as primers. A gene usable as a template in this PCR can include, for example, cDNA prepared from various cell strains. Using restriction enzymes, the reporter gene prepared in this manner is inserted into a commercial vector, and a gene encoding the epitope tag is similarly integrated downstream of the protein-coding gene on the vector, whereby a reporter plasmid can be prepared.

A gene (SEQ ID NO: 15) encoding a fusion protein in which an epitope tag (SEQ ID NO: 3) consisting of 19 residues having 2 epitopes was fused with human spermidine synthase (SPDS, GenBank Accession No. NP003123) was used in the Examples. The spermidine synthase is an enzyme that synthesizes spermidine as one kind of polyamine serving as a substrate in nucleic acid synthesis.

### Epitope

It is important that for the epitope used in the present invention, its amino acid sequence be specified. As the sequence of the epitope, an amino acid sequence known as an epitope can be utilized, or the sequence may be originally designed. The sequence used is usually a part of a certain protein not binding to those proteins occurring in a cell to induce expression. The epitope is composed preferably of 6 to 30 amino acid residues, more preferably 6 to 8 amino acid residues. For example, FLAG (DYKDDDDK^{™} manufactured by Sigma), c-myc (EQKLISEEL), polyhistidine (HHHHHH) etc. are epitopes known to those skilled in the art, and antibodies capable of binding specifically to these epitopes are easily commercially available. Epitopes having such amino acid sequence can be used as the epitopes of the present invention even if they have any amino acids before and after the amino acid sequence. When an amino sequence is originally designed, the resulting epitope can be used as the epitope of the present invention by confirming, by methods such as those described later, that it has a property of binding specifically to the antibody.

To carry out the method of the present invention, a method of specifying an antibody epitope includes, but is not limited to, (A) a method of determining an epitope by specifying an amino acid sequence binding specifically to a certain antibody and (B) a method of using an antibody prepared by immunization with a specific peptide antigen.

The method (A) of determining an amino acid sequence binding specifically to a certain antibody includes a method of specifying it by western blotting with the antibody (Proc. Natl. Acad. Sci. U.S.A. 76, 3116 (1979)). Specifically, when a protein for example is used as the antigen, a DNA encoding the protein is cut with restriction enzymes etc. into fragments each encoding about 50 to 200 amino acids, and each DNA fragment is inserted into a suitable expression vector which is then transcribed and translated in a suitable host to express a protein, and the ability of the protein to bind to the antibody is examined. The suitable expression vector may be any vector compatible with a host into which it is introduced. For example, when *Escherichia coli,* yeasts, animal cells and insect cells are used as hosts, pET, pNMT, p cDNA and pFastBac (all available from Invitrogen) can be used respectively. As a system for transcription and translation, a cell-free transcription/translation system prepared from rabbit reticulocytes, an extracted wheat germ, an extract from *Escherichia coli* (*E*. *coli* S30 extract) or the like can also be used. The peptide fragments as epitope candidates can thereby be reduced to a smaller number of certain peptide fragments, and then DNA fragments each encoding about 5- to 50-amino acid sequences, out of the peptide fragments, are prepared by polymerase chain reaction (PCR) or from synthetic oligonucleotides, and then expressed as fusion proteins fused with a suitable protein, and the ability thereof to the antibody is examined. The suitable protein used in the fusion protein may be any protein not binding to the antibody to be analyzed. In this manner, the epitope that is a polypeptide as the smallest unit necessary for binding to the antibody can be specified.

On the other hand, the method (B) of using an antibody prepared by immunization with a specific peptide antigen is a method wherein a peptide having a specific amino acid sequence is used as an antigen in advance of antibody production, and also used in screening for the antibody. Because the epitope of the antibody thus prepared has been previously determined, the above peptide can be identified as the epitope of the antibody.

As a method of preparing an antibody specifically recognizing a specific amino acid sequence contained in a certain protein, it is possible to use, for example, a method described in Antibodies: A Laboratory Manual (1989) (Cold Spring Harbor Laboratory Press). This method is specifically as follows. First, an immunogen is necessary for preparing the antibody. The term "immunogen" as used herein refers to a substance having an ability to generate or induce an immune response in a living body. The immunogen can be produced according to a method known per se or a method pursuant thereto. The immunogen is used as a conjugate with a carrier protein such as bovine serum albumin (BSA), bovine thyroglobulin (BTG) or keyhole limpet hemocyanin (KLH) as necessary. Usually, a peptide does not have an immune response because of its lower molecular weight, and therefore, such conjugate is desirably used as the immunogen.

Immunization can be carried out by administering the immunogen to a mammal via intravenous, intradermal, subcutaneous or intraperitoneal injection or the like. More specifically, the immunogen is diluted to an appropriate concentration for example with phosphate buffered saline (PBS), physiological saline and the like, and administered to a test animal 3 to 10 times in total at two- to six-week intervals in combination with a usual adjuvant according to need. As the mammal to be immunized with the immunogen, rabbit, goat, sheep, mouse, rat and the like are generally used. When a mouse is used, a dosage is about 50 µg per mouse. The "adjuvant" refers to a substance which nonspecifically enhances an immune response to an antigen when administered together with the antigen. Examples of an adjuvant usually used include whooping-cough vaccine, Freund's adjuvant and the like. By collecting blood from a mammal three to ten days after the last immunization, a polyclonal antibody can be obtained.

A method for producing a monoclonal antibody can be carried out by preparing fusion cells (hybridoma) between plasma cells (immunocyte) of an immunized mammal with an immunogen and plasmacytoma cells (myeloma cells) of the mammal, then selecting clones which produce a monoclonal antibody recognizing a desired antigen, and culturing the clones. The monoclonal antibody can be produced essentially according to the standard methods (see Kohler, G. and Milstein, C., Nature, 256, 495-497 (1975)).

In the above method, the immunized mammal is preferably selected in view of the compatibility with plasmacytoma cells used in cell fusion, and mouse and rat are used for such purpose.

A hybridoma can be obtained from the resultant immunocyte according to, for example, the method described in "Experimental Manual for Molecular Cell Biology" (Takekazu Horie et al., 1994, Nankodo), with the aim of producing cells which can be subcultured, by fusing the immunocyte producing an antibody with plasmacytoma cells in the presence of polyethylene glycol. Plasmacytoma used in the method is preferably derived from the same homothermal animal species among homothermal animals. For example, when fusing with spleen cells obtained from an immunized mouse, mouse myeloma cells are preferably used. As plasmacytoma cells, known cells such asp3×63-Ag8.UI may be used.

A hybridoma can be selected by culturing the fused cells in a HAT medium (hypoxanthine-, aminopterin- and thymidine-added medium). A hybridoma producing an objective antibody can be obtained by examining (screening for) the binding of an antibody secreted in a culture supernatant to an antigen in the stage of a colony being ascertained. A method for screening, which is exemplified by a variety of methods generally used for detecting an antibody, such as spotting, agglutination reaction, western blotting and ELISA, is preferably conducted according to ELISA utilizing reactivity to an antibody as an indicator for a culture supernatant of hybridoma. By the method of screening, a cell line producing a desired antibody which reacts specifically to an antibody can be screened.

Cloning of the cell line producing a desired antibody obtained by screening can be conducted according to usual limiting dilution and the like. The cloned hybridoma may be cultivated in a large scale in serum-added medium or serum-free medium according to need. According to the cultivation, a desired antibody of relatively high purity can be obtained as a culture supernatant. The desired antibody can be recovered abundantly as murine ascites by inoculating a hybridoma intraperitoneally into a mammal such as mouse having compatibility to the hybridoma.

A culture supernatant and murine ascites containing the hybridoma which produces the antibody of the present invention may be used as a crude antibody solution without purification or modification. Alternatively, these may be purified by conventional methods such as ammonium sulfate fractionation, salt precipitation, gel filtration, ion exchange chromatography, affinity chromatography and the like.

A polypeptide containing amino acid sequences set forth in SEQ ID NOS: 1 and 2 in the Sequence Listing, which is derived from human type 2 collagen previously identified by Downs et al. (Journal of Immunological Methods, 247 (2001)), was used as the epitope used in the Examples of the invention. A monoclonal antibody specifically recognizing the epitope was prepared according to the method described in this specification.

Specific examples of the thus prepared antibodies that can be used in the present invention include, for example, a monoclonal antibody (6G4 antibody) which as an antibody binding specifically to a polypeptide having the amino acid sequence set forth in SEQ ID NO: 1, was produced by hybridoma TAG-6G4 and a monoclonal antibody (2E6 antibody) which as an antibody binding specifically to a polypeptide having the amino acid sequence set forth in SEQ ID NO: 2, was produced by hybridoma TAG-2E6. These monoclonal antibodies have been deposited with International Patent Organism Depositary in National Institute of Advanced Industrial Science and Technology (AIST) (Tsukuba Central 6,1-1-1 Higashi, Tsukuba, Ibaraki, Japan) since January 13, 2006, as "Mouse-Mouse Hybridoma TAG-2E6" under the accession number FERM ABP-10482 and "Mouse-Mouse Hybridoma TAG-6G4" under the accession number FERM ABP-10483 under the Budapest Treaty on the International Recognition of the Deposit of Microorganisms for the Purposes of Patent Procedure.

These antibodies possess the following physicochemical and immunological properties:
6G4 antibody
(a) Specifically binding to a peptide consisting of an amino acid sequence
   GEPGDDAPS.
(b) It is a 150-kDa protein consisting of an H chain of 50-kDa and an LH chain of 27 kDa, having an H-chain variable region (VH region) represented by SEQ ID NO: 4 and an L-chain variable region (VL region) represented by SEQ ID NO: 5.
(c) Belonging to immunoglobulin class TgG1 (k).
   2E6 antibody
(d) Binding specifically to a peptide consisting of an amino acid sequence GPPGPQG.
(e) It is a 150-kDa protein consisting of an H chain of 50-kDa and an LH chain of 27 kDa, having a VH region represented by SEQ ID NO: 6 and a VL region represented by SEQ ID NO: 7.
(f) Belonging to immunoglobulin class IgG2b (k).

### Cell

The cell used in the method of the present invention is a cell having the above reporter plasmid introduced into a usual animal cell or the like as a host cell. The usable host cell includes, for example, mammalian cells derived from humans, mice, rats etc., amphibian cells derived from frogs etc., and insect cells, etc. Cells capable of stable subculture are preferable in consideration of operativity, reproducibility, etc. Specific examples of preferable host cells include, for example, human-derived 293 cell, human-derived A-431 cell, human-derived HeLa cell, human-derived nuroblastoma cell, mouse-derived NIH3T3 cell, hamster-derived CHO-K1 cell, monkey-derived COS-1 cell, and rat-derived L6 cells.

Introduction of the reporter plasmid into a host cell can be conducted by general methods of DNA transfer (transfection) such as electroporation, the calcium phosphate method, and lipofection, insofar as each gene contained in the plasmid can function in the cell. A cell strain maintaining the reporter plasmid stably can also be selected if a drug resistance gene has been added to the reporter plasmid.

The medium composition used in preparing the cells used in the method of the present invention, cell culture conditions, and transfection conditions for each vector plasmid, can be the same as for this kind of host cell and in conventional methods utilizing a gene delivery vector.

A cell inherently having genes encoding constituent factors (a receptor, a ligand, a transcription factor, a coactivator etc.) necessary for transcription reaction in the cell can be used in the assay of the present invention by introducing only the above-mentioned reporter plasmid to the cell. However, a cell not inherently having genes encoding constituent factors necessary for transcription reaction may be endowed with an ability to express the above gene by introduction (co-transfection) of desired and necessary genes accordingly.

As receptors involved in transcription, there are known not only receptors present on cell surfaces (cell membrane receptors) such as G-protein-coupled receptor (GPCR), an enzyme-type receptor (tyrosine kinase type, serine/threonine kinase type, guanylate cyclase), cytokine receptors and ion channel type receptors, but also intracellular receptors including steroid hormone receptors such as an androgen receptor (GenBank Accession No. M23263) and an estrogen receptor (GenBank Accession No. X03635), and a nitric oxide (NO) receptor.

Generally, the cytokine receptor molecule itself does not show a tyrosine kinase activity, and a non-receptor type tyrosine kinase called Janus kinase (JAK) occurring in the vicinity of the receptor is activated to emit a first signal into a cell. Upon receipt of this signal, other signaling molecules such as STAT (signal transducer and activator of transcription protein) are phosphorylated.

For example, IL-4 shown in Example 4 below is known as a cytokine involved in induced expression of MHC class II, CD23 antigen and IL-4 receptor a, in class switch of B cells, and in induced differentiation of helper T cells, and when IL-4 is bound to receptors occurring on cell membranes, the receptors are dimerized followed by intracellular activation of JAK1 and JAK3 that are tyrosine kinases, to phosphorylate IL-4 receptors. Then, a transcription factor STAT6 is led to a phosphorylation site of the receptor and phosphorylated by JAK. It is known that phosphorylated STAT6 forms a dimer, moves to a nucleus, and binds to a recognition sequence on DNA thereby activating a promoter, to induce the expression of a gene involved in cell response.

Transcriptional regulatory activity via IL-4 regulatory element contained in germ line ε promoter region of immunoglobulin H chain is examined in the Examples shown later. In IL-4 regulatory element, a recognition sequence of STAT6, and a binding region of CCAAT-enhancer binding protein (C/EBP) next thereto, are present (SEQ ID NO: 16). It is known that STAT6 located downstream of an intracellular signal transduction system of IL-4 binds to the element thereby regulating transcription of a gene downstream thereof and participates in terminal differentiation of B cell and in class switch of antibody. Accordingly, when this transcriptional regulatory mechanism is disturbed by some factors, functions such as antigen-specific activation of B cell and class switch become insufficient, humoral immunodeficiency is caused, and allergic diseases and autoimmune diseases may be caused.

Accordingly, it was conceivable that by regulating this transcriptional function, it is possible to prevent and treat immunodeficiency diseases (for example, IgA nephropathy, hypogammaglobulinemia, hyper-IgM-emia, etc.), autoimmune diseases or allergies, attributable to dysfunctions of B cell or insufficiency in class switch of immunoglobulin, and also that low-molecular compounds inhibiting the transcriptional regulatory mechanism by 1L-4 receptor-mediated intracellular information transmission can be pharmaceutical preparations for treating such diseases.

Some intracellular receptors form complexes with a ligand in a cell and then bind to a specific recognition sequence, thereby promoting transcription of a gene downstream thereof. As the ligand, glucocorticoid (Nature, 318, 635-641 (1985)), estrogen, and dioxin ( J. Biol. Chem., 263, 17221-17224 (1988)), etc. are known. When a transcriptional inhibitory (antagonist) activity on these receptors is measured, a gene encoding the ligand may be introduced into a cell to confer its expression ability on the cell.

A DNA of a gene sequence of a factor involved in such transcription reaction can be prepared by PCR wherein oligonucleotides for the coding DNA, which are designed and prepared on the basis of a known nucleotide sequence, are used as primers. The DNA used as a template in such PCR can include commercially available cDNAs derived from various organisms.

When the exogenous gene is introduced into a host cell, the gene is inserted into a vector such that the gene is linked operatively downstream of a suitable promoter, and the resulting vector is introduced into a cell.

The promoter is a promoter capable of functioning in a cell into which the vector is introduced, that is, a promoter having an ability to initiate transcription; for example, when the cell is a eukaryotic cell, examples of the promoter include Rous sarcoma virus (RSV) promoter, cytomegalovirus (CMV) promoter, simian virus (SV40) early or late promoter, etc. The vector can be endowed with a drug resistance gene for selecting an objective cell. The method of introducing such vector into a host cell can be carried out according to the above method of introducing the reporter plasmid.

### Step of Contacting with Test Substance

The method of the present invention comprises a step of contacting the thus prepared cell with a test substance. The test substance can be contacted with the cell by adding it to a cell culture. Alternatively, when the test substance is a protein, the test substance can be contacted with the cell by allowing the cell to express a gene encoding the protein.

### Step of Contacting with Detection Antibodies

The method of the present invention further comprises a step of the above cell with detection antibodies. As used herein, detection antibodies refer to antibodies labeled with labeling substances in order to detect closeness of the antibodies to each other. These detection antibodies may be added to a medium in which a cell is then cultured, or may be added after conclusion of culture. The antibodies may be either monoclonal or polyclonal insofar as they specifically recognize a polypeptide having an amino acid sequence containing the epitope, but a monoclonal antibody is desirable. The antibody can be prepared in a manner similar to the method described above. The antibody can be labeled according to conventional methods such as described in "Experimental Manual for Molecular Cell Biology" (Takekazu Horie et al., 1994, Nankodo) or by manuals attached to labeling substances.

Examples of the labeling substances include a luminescent substance, an enzyme, a fluorescent substance, beads, a radioisotope, a metal, biotin and the like. The luminescent substance refers, for example, to chemiluminescent substances such as lucifenol, luminol, aequorin, and acridinium ester. The enzyme refers, for example, to luciferase, β-galactosidase, alkaline phosphatase and peroxidase. The fluorescent substance refers, for example, to lanthanides such as europium (Eu) and terbium (Tb), lanthanide derivatives such as europium cryptate, fluorescein derivatives such as fluorescein isothiocyanate (FITC), rhodamine derivatives such as tetramethylrhodamine isothiocyanate (RITC), and fluorescence proteins such as YFP, GFP, CFP, BFP and allophycocyanin. The beads refer, for example, to beads subjected to special treatment, such as protein A beads, wheat germ agglutinin (WGA) beads and streptavidin beads. The radioisotope refers, for example, to ¹⁴C, ¹²⁵I, ³H and ³⁵S, and also encompasses compounds labeled therewith. The metal refers, for example, to ferritin and colloidal gold.

When labeling substances are selected, a combination of labeling substances capable of detecting a phenomenon accompanying the closeness of the detection antibodies to each other should be selected. Such combination generally known in the art includes, for example, a combination of a fluorescence substance label and a fluorescence substance label, a luminescence substance label and a fluorescence substance label, or a radioisotope label and beads.

The "step of contacting with the detection antibodies recognizing epitopes" refers to addition of the detection antibodies capable of immune-specific recognition of 2 epitopes (first and second epitopes) respectively contained in a reporter protein, to a reporter protein-containing cell sample. The detection antibodies are added usually after dilution with a suitable buffer (for example, Tris-buffered physiological saline containing 0.8 M potassium fluoride and 0.5% bovine serum albumin). The dilute concentration used can be determined by confirming that the concentration leads to the conditions under which the "phenomenon accompanying the closeness of the detection antibodies to each other" (to be described later) by preliminary examination can be efficiently detected. For example, when the "phenomenon accompanying the closeness of the detection antibodies to each other" is fluorescence resonance energy transfer (FRET), the concentration of the detection antibody used in the assay can be determined for example by confirming its efficiency by the fluorescence intensity of the acceptor.

### Step of Detecting Phenomenon Caused by Both the Detection Antibodies Coming Close to Each Other and Correlating the Detected Phenomenon with the Effect of the Test Substance on Transcriptional Regulatory Mechanism

The method of the present invention comprises detecting a phenomenon caused by both the detection antibodies coming close to each other after contacting the cell with the detection antibodies, and then correlating the detected phenomenon with the effect of the test substance on transcriptional regulatory mechanism. The "detection of the phenomenon caused by both the detection antibodies coming close to each other" refers to detection of the phenomenon caused by two detection antibodies' labels coming close to each other when the detection antibody recognizing the first epitope binds to the first epitope and the detection antibody recognizing the second epitope binds to the second epitope. The method of detecting the interaction between two proteins is well-known to researchers in this field and includes, for example, the BRET method, the FRET method, AlphaScreen (amplified luminescent proximity homogeneous assay^{™}), SP A (Scintillation Proximity Assay^{™}) etc.

The above phenomenon can also be related to the effect of a test substance on the transcriptional regulatory mechanism. In the method of the present invention, the degree of detectable value reflects the degree of transcriptional regulatory activity. Accordingly, the effect (transcriptional promoting activity, transcriptional inhibitory activity etc.) of the test substance on the transcriptional regulatory mechanism can be evaluated by detecting the phenomenon caused by both the detection antibodies coming close to each other.

In a preferable mode, the method of detecting the phenomenon caused by both the detection antibodies coming close to each other comprises detecting excitaion energy transfer due to resonance. For example, when a fluorescence substance is irradiated with an exciting light, the fluorescence substance is excited to emit its energy as fluorescence or heat energy and then returns to the ground state (extinction). At this time, another fluorescence substance, if occurring in the proximity of the above fluorescence substance, receives its energy and is excited thereby similarly showing a phenomenon of emitting fluorescence. Such phenomenon is known as fluorescence resonance energy transfer (referred to hereinafter as FRET). The fluorescence intensity is measured with a measuring instrument such as a spectrofluorometer, whereby the effect of a test substance on the transcriptional regulatory mechanism can be measured.

In this case, a method of selecting a fluorescence substance having a longer fluorescence life (longer time until extinction) as a donor (molecule providing energy) can be mentioned as a particularly preferable mode. In this method, the fluorescence of interfering substances such as a cell sample and plastics is short-lived, and thus the influence of background fluorescence caused by interfering substances can be eliminated by measurement after a predetermined time after excitation. Such FRET is called time-resolved fluorescence resonance energy transfer (TR-FRET). A method of detecting TR-FRET includes HTRF (Homogeneous Time-Resolved Fluorescence, CIS Bio International), LANCE (Perkin Elmer Life Science) etc.

HTRF is characterized by using two fluorescence substances. The two fluorescence substances are specifically a europium compound that is europium cryptate (hereinafter referred to as cryptate, having a trisbipyridine cage structure coordinated with a europium ion of rare earth element) and an allophycocyanin derivative XL665 (stabilized by crosslinking 3 molecules of allophycocyanin that is a fluorescent protein originated from blue-green algae). Cryptate, upon irradiation with an exciting light at 337 nm, will emit long-lived fluorescence at 620 nm, but if XL665 becomes adjacent to the cryptate when the antibodies come close to each other, the excitation energy transfers by FRET to XL665, and XL665 will in turn emit long-lived fluorescence at 665 nm. By measuring this long-lived fluorescence, the reporter protein can be determined. The advantage of this method is that by measuring fluorescence after a predetermined time after excitation of cryptate, only long-lived (up to 1 millisecond) fluorescence can be selectively detected by eliminating the influence of short-lived (up to 10 nanosecond) background fluorescence caused by fluorescent substances contained in a measurement sample and a measurement tube. By simultaneously measuring the fluorescence of XL665 at 665 nm and the fluorescence of cryptate at 620 nm, the measurement value is expressed as a ratio value of (fluorescence intensity at 665 nm/fluorescence intensity at 620 nm)×10,000, thereby compensating for the change in measurement value caused by a varying amount of added reagents or by the color quenching effect (inner filter effect) of a measurement sample.

In another mode, a detection method of using bioluminescence resonance energy transfer (BRET) can also be mentioned. For example, antibodies labeled respectively with luciferase and a green fluorescence protein variant (GFP variant) are used as the two detection antibodies. When these antibodies come close to each other, there occurs a phenomenon (BRET) wherein a part of the energy generated by the luminescence reaction between luciferin and luciferase transfers to the GFP variant to emit fluorescence. By measuring the fluorescence intensity at this time, the effect of a test substance on the transcriptional regulatory mechanism can be determined.

In another mode, a method of using a combination of detection antibodies labeled respectively with beads (for example, SPA beads) and a radioisotope (for example, ³H, ¹⁴C, ¹²⁵I etc.) is used. When the two detection antibodies are come to close to each other, radiations such as β-ray emitted by the radioisotope reach scintillators in the beads to generate a luminescence phenomenon, and by detecting the luminescence, the effect of a test substance on the transcriptional regulatory mechanism can be measured.

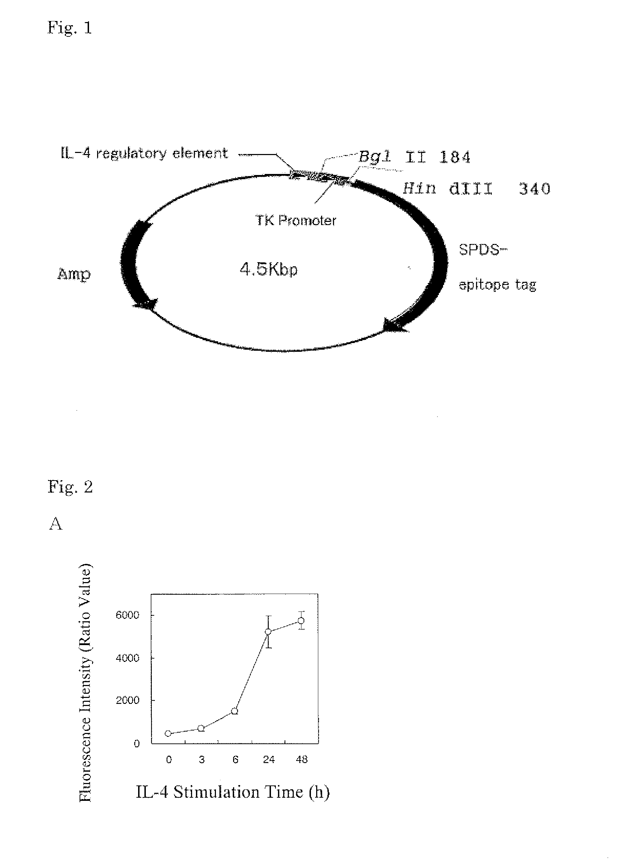

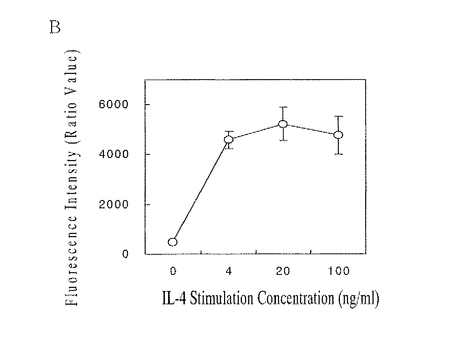

Examples detectable by the method of the present invention can include an interleukin 4 (IL-4)-mediated intracellular signaling system. An IL-4 regulatory element of germ line ε promoter for immunoglobulin H chain (T. Mikita et al. Mol. Cell. Biol. 1996, pp. 5811-5820, SEQ ID NO: 16) and a thymidine kinase promoter (SEQ ID NO: 17) of phRL-TK vector (Promega) were integrated in an upstream region of a luciferase gene of pGL3 basic vector (Promega), and the luciferase gene was replaced by the reporter gene of the present invention (that is, a gene (SEQ ID NO: 15) having an epitope tag linked with SPDS), thereby preparing a reporter plasmid. An outline of the reporter plasmid is shown in Fig. 1. Using cells obtained by introducing this plasmid into HeLa cells derived from human epithelial cancer, the correlation of the concentration of IL-4 added and the time of stimulation with IL-4, with the detection value, was determined according to the method of the present invention. As a result, it could be confirmed that the fluorescence intensity is changed depending on the stimulating concentration and stimulation time with IL-4. This indicates that the promotion of transcription by intracellular signal transduction starting from IL-4 can be detected by the method of the present invention.

### Reporter Gene Assay

The working mode of the reporter gene assay of the present invention is essentially the same as this type of reporter gene assay known in the art, and it can also be said that the screening method is in one mode thereof.

Specifically, the cell of the present invention is seeded and cultured on a cell culture vessel. For example, when a 96-well plate is used, usually about 10⁴ to 10⁵ cells are seeded per well and cultured for about 1 hour to overnight. Then, a test substance is added to the cell culture. The test substance may be any substance such as a peptide, a protein, a non-peptidic compound, a synthetic compound (for example a low molecular weight compound), a fermentation product, a cell extract, a plant extract, an animal tissue extract and the like, or may be a solution containing the same. When the transcriptional promoting activity of a test substance is measured, a solution containing a test substance dissolved in a solvent, or a solvent only, is added to the cell culture at a final concentration of the solvent usually in the range of about 0.1 to 2% in the culture. When the transcriptional inhibitory activity of a test substance is measured, a system wherein a solution containing a substance having a receptor ligand activity dissolved in a medium or the like is added to the above culture such that the concentration of the ligand in the culture becomes usually about EC50, or a system wherein a test substance is further added to the above system, is prepared. As the solvent, dimethyl sulfoxide (DMSO), ethanol etc. are often used.

For example, if cells in the system to which a test substance was added showed a higher detection value per cell than that of cells in the system to which the solvent only was added, in a test for measurement of the transcriptional promoting activity of a test substance, then the test substance is judged to exhibit a transcriptional promoting activity. If cells in the system to which the ligand and a test substance were added showed a lower detection value per cell than that of cells in the system to which the ligand only was added, in a test for measurement of the transcriptional inhibitory activity of a test substance, then the test substance is judged to exhibit a transcriptional inhibitory activity.

Hereinafter, the present invention is described in more detail by reference to the Examples, but the present invention is not limited thereto.

### Example 1

### Preparation of Reporter Plasmid

A thymidine kinase promoter (TK promoter, SEQ ID NO: 17) of phRL-TK vector (manufactured by Promega) was amplified by PCR and inserted via restriction enzyme sites (BglII and HindIII) into pGL3-Basic vector (manufactured by Promega) (pGL3-TK). Then, an IL-4 regulatory element (SEQ ID NO: 16) was synthesized and polymerized by T4 polynucleotide kinase, and its tetramer fragment was separated by agarose gel electrophoresis. The tetramer fragment was inserted via a restriction enzyme site (BglII) into a region upstream of TK promoter of pGL3-TK. The product was further digested with restriction enzymes Ncol and Xbal, and its DNA fragment (3.4 kb) excluding a luciferase gene was purified by agarose gel electrophoresis. Total RNA was purified from U2OS cells (human osteosarcoma cell strain, American Type Culture Collection), and a gene (SEQ ID NO: 15) having an epitope tag gene (SEQ ID NO: 14) ligated to SPDS (GenBank Accession Number BC000309, a human working draft contig identifier NM_003132) was amplified by RT-PCR. The PCR product was inserted via restriction enzyme sites (Ncol and XbaI) into the 3.4-kb DNA fragment, to prepare a reporter plasmid (Fig. 1).

### Example 2

### Method for Preparing Antibody

### (1) Preparation of 6G4 Antibody

A polypeptide consisting of an amino acid sequence having cysteine added to the C-terminal side of a region (SEQ ID NO: 1) corresponding to amino acids in positions 757 to 765 in human type 2 collagen was conjugated with keyhole limpet hemocyanin (KLH, Pierce) via sulfosuccinimidyl 4-(N-maleimidomethyl)-cylohexane-1-carboxylate (sulfo-SMCC, Pierce), to yield an immunogen. The immunogen was mixed with Freund's complete adjuvant (Difco) to give an emulsion, and 40 µl of the emulsion was administered intraperitoneally into a mouse (Balb/c, CrSlc, 6-week-old, female) at 3-week intervals. The spleen was excised from the mouse after immunized 4 times, and then fused by the PEG method with myeloma cells (p3×63-Ag8.UI, Tokyo Cancer Institute) to prepare a hybridoma. On the ninth day of culture, the culture supernatant was collected and screened for a positive well containing an antibody-producing hybridoma. Screening was carried out by time-resolved fluorescence immunoassay (DELFIA, Amersham) described below. 50 µl measurement buffer (50 mM Tris-HCl buffer (pH 7.5) containing 150 mM Nad, 0.01% Tween 80, 0.5% BSA, 0.05% NaN₃), 50 µl of the culture supernatant, and 50 µl labeled antigen (measurement buffer containing 20 ng/ml biotin-labeled antigen peptide and 100 ng/ml europium-labeled streptavidin (PerkinElmer Life Science)) were added successively onto a microplate (Sumitomo Bakelite Co., Ltd.) having anti-mouse IgG antibody (Shibayagi) immobilized thereon, and then incubated at 4°C for 16 hours. After the plate was washed twice with 200 µl wash (150 mM NaCl, 0.02% NaN₃, 0.01% Tween 20), 150 µl enhancement solution (Perkin Elmer Life Science) was added to each well which was then measured for time-resolved fluorescence with a 1420ARVOsx multi-label counter (Perkin Elmer Life Science). In this screening, one highly active well was selected and subjected twice to cloning by limiting dilution to establish an antibody-producing hybridoma TAG-6G4. When a subclass of TAG-6G4 was examined by a mouse monoclonal antibody isotyping ELISA kit (BD Bioscience), it was IgG 1(k). Hybridoma TAG-6G4 was administered intraperitoneally into a nude mouse (BALB/cANNCrj-nu-nu) from which ascites was then collected (entrusted to Laboproducts Inc.). From the ascites, a purified antibody was obtained by affinity chromatography on a protein A column.

### (2) Preparation of 2E6 Antibody

A polypeptide consisting of an amino acid sequence having cysteine added to the N-terminal side of a region (SEQ ID NO: 2) corresponding to amino acids in positions 769 to 775 in human type 2 collagen was conjugated with bovine serum albumin (BSA, Pierce) via (N-e-maleimidocaproyloxy) sulfosuccinimide ester (sulfo-EMCS, Pierce), to yield an immunogen. The immunogen was administered in the same manner as for TAG-6G4 into a mouse (A/J Jms Slc, 6-week-old, female). The spleen was excised from the mouse and then subjected to cell fusion. Screening by DELFIA and cloning were carried out to establish an antibody-producing hybridoma TAG-2E6. A subclass of the antibody was IgG2b (k). Ascites formation in a nude mouse and subsequent affinity chromatography on a protein A column gave a purified antibody.

### (3) Determination of Amino acid Sequences of VH and VL Regions

Using Rneasy Mini kit (QIAGEN), total RNA was purified from 1×10⁷ hybridomas. Using SMART RACE cDNA (BD Bioscience), gene sequences of the VH and VL regions were determined according to a protocol of the kit. An SM ARTII A oligonucleotide attached to the kit, a primer (SEQ ID NO: 8) specific to the VH region of TAG-6G4, a primer (SEQ ID NO: 9) specific to the VH region of TAG-2E6, and a primer (SEQ ID NO: 10) specific to the VI region, were used to synthesize cDNA. The VH and VL genes were amplified by PCR wherein the cDNA was used as a template together with a universal primer attached to the kit, a primer (SEQ ID NO: 11) specific to the VH region of TAG-6G4, a primer (SEQ ID NO: 12) specific to the VH region of TAG-2E6, and a primer (SEQ ID NO: 13) specific to the Vl region. The PCR products were cloned by a TOPO TA cloning kit (Invitrogen) and analyzed for their sequences (entrusted to Operon Biotechnology). From the resulting gene sequences, amino acid sequences of the VH and VL regions were determined.

### Example 3

### Method for Preparing Detection Antibodies

### (1) Labeling 6G4 Antibody with Cryptate

6G4 antibody was dissolved at a concentration of 1 mg/ml in 0.1 M phosphate buffer (pH 8.0). 15 moles of cryptate TBP monosuberate (CIS Bio International) was added to 1 mole of the antibody and incubated at 25°C for 1 hour. The reaction solution was subjected to gel filtration through a PD-10 column (Amersham) previously equilibrated with PBS, to fractionate the labeled antibody. 0.1% bovine serum albumin, 0.1% Tween 20, and 0.05% sodium azide were added to the labeled antibody which was then stored at -70°C.

### (2) Labeling 2E6 Antibody with XL665

To 2E6 antibody dissolved in 0.1. M phosphate buffer (pH 7.0) was added an 8-fold molar excess of N-succinimidyl 3-(2-pyridylthio)propionate (SPDP, Pierce), followed by incubation at 25°C for 20 minutes. 10 mM dithiothreitol (DTT) was added at a final concentration of 10 mM to the reaction solution which was then incubated for additional 10 minutes, followed by gel filtration through a PD-10 column previously equilibrated with 0.1 M phosphate buffer (pH 7.0) containing 10 mM EDTA, to fractionate 2E6 antibody having a cysteine group introduced into it (SH-2E6 antibody). XL665 (CIS Bio International) was diluted in 0.1 M phosphate buffer (pH 7.0), and a 5-fold molar excess of Sulfo-SMCC was added thereto, and the mixture was reacted at 25°C for 30 minutes. By gel filtration with a PD-10 column previously equilibrated with 0.1 M phosphate buffer (pH 7.0), XL665 having a maleimide group introduced into it was fractionated (maleimidated XL665). To 1 mole of the maleimidated XL665 were added 3 moles of the above SH-2E6 antibody, and the mixture was reacted at 4°C for 16 hours to give XL665-labeled antibody. To the labeled antibody was added a 100-fold molar excess of N-ethylmaleimide, and then the mixture was left at room temperature for 10 minutes, and 0.1% bovine serum albumin, 0.1% Tween 20 and 0.05% sodium azide were added thereto, and the resulting antibody was stored at -70°C.

### Example 4

### (1) Preparation of Cells in the Reporter Assay of the Invention

HeLa cells (cells deposited with ATCC) were dispersed at a density of 1.2×10⁵ cells/ml in a medium (MEM medium containing 10% fetal bovine serum, 100 units/ml penicillin G, 100 µg/ml streptomycin) and then put in a volume of 100 µl per well on a 96-well tissue culture plate. After 24 hours, 20 µg of the reporter plasmid prepared in Example 1, and 60 µl FuGENE6 (Roche), were added to 2 ml FBS-free medium, then left at room temperature for 20 minutes, and added in a volume of 5 µl to each well, followed by incubation for 24 hours to transfect the cells with the reporter plasmid.

### (2) Confirmation of Transcriptional Regulatory Activity

After the culture supernatant of the cells was removed, 100 µl of IL-4 diluted with a medium was added at final concentrations of 0, 4, 20 and 100 ng/ml respectively to the respective wells and cultured for 3, 6, 24 and 48 hours. After culture, a cell lysis buffer (Tris buffered physiological saline containing 1% Triton X-100, 5 mM EDTA and a protease inhibitor cocktail) was added in a volume of 100 µl to each well and left at room temperature for 1 hour. 10 µl of the supernatant on each well was transferred onto a 384-well assay plate (black, low-volume type, Corning), and 10 µl of an assay buffer containing the 2 detection antibodies (Tris buffered physiological saline containing 20 ng/ml cryptate-labeled 6G4 antibody, 5,000 ng/ml XL665-labeled 2E6 antibody, 0.8 M potassium fluoride, and 0.5% BSA) was added to each well, followed by incubation at room temperature for 24 hours. After the reaction was finished, the fluorescence intensity of each well at 665 nm was measured with a time-resolved fluorescence microplate reader (Rubystar, BMG Labtechnologies). The measurement value was expressed as the ratio value to the fluorescence intensity of cryptate at 620 nm.

The results are shown in Fig. 2. The cells into which the reporter plasmid had been introduced showed an increasing ratio value depending on the concentration of IL-4 and the stimulation time with IL-4. This increase in ratio value indicates that the expression of the reporter molecule (SPDS-epitope fragment) is enhanced by intracellular signal transduction attributable to IL-4, and it can be said that as a result of binding of activated STAT6 to the IL-4 regulatory element, the transcription is activated.

An about 10-fold increase in ratio value by stimulation with IL-4 indicates that the method of the present invention can also be applied to high through-put screening intended to develop pharmaceutical preparations. For example, HeLa cells are transfected in the same manner as in (1) above, then the culture supernatant is removed, then 90 µl of a new medium is added to each well, and a test substance prepared at a concentration of 0.2 to 1 mg/ml in a suitable solvent is added in a volume of 1 µl to each well, followed by pre-incubation for 30 minutes. Then, 10 µl medium containing 40 ng/ml IL-4 is added to each well and then incubated for 24 hours, and the fluorescence intensity is measured in the same manner as in (2) above. By confirming that the fluorescence intensity is significantly lower than in the absence of a test substance, it is possible to detect a test substance having an inhibitory activity on the transcriptional regulatory mechanism by the IL-4-mediated intracellular information transduction.

### Example 5

### Comparison Between HTRF Method and Luciferase Assay

### (1) HTRF Method

20 µl HeLa cells (2×10⁴ cells) transfected in the same manner as in Example 4 were added to each well of a 384-well plate (No. 3709, Corning) to which 1 µl of 10% DMSO solution containing 0.15 mg/ml test compound dissolved therein had been previously added, then the cells were cultured for 30 minutes, and 10 µl medium containing 3 ng/ml IL-4 was added to each well, followed by culture for additional 24 hours. After culture, 30 µl of the HTRF reagent was added to each well which was then incubated at room temperature overnight, and the HTRF of each well was measured by Rubystar. The transcriptional inhibitory activity of the test compound was calculated assuming that the measurement value obtained from the cells treated with IL-4 without adding the compound was 0%, while the measurement value obtained from the cells not treated with IL-4 was 100%.

### (2) Luciferase Assay (Chemiluminescence Method)

The fragment consisting of mTK and four IL-4 regulatory elements ligated therein, described in Example 1, was inserted via BgIII and HindIII restriction enzyme sites into pGL 3-Basic vector to give a reporter vector for luciferase assay. First, HeLa cells into which this vector had been stably introduced were screened (HL-10 cells). 1 µl of the above test compound solution and 20 µl of HL-10 cells (2×10⁴ cells) were added to each well of a 384-well plate (No. 3704, Corning) and cultured for 30 minutes, then 10 µl medium containing 3 ng/ml IL-4 was added to each well, and the cells were cultured for 24 hours. After culture, 30 µl PicaGene (Toyo Ink) was added to each well and incubated at room temperature for 1 hour, and the luminescence of each well was measured with a multi-label counter (ARVO). The transcriptional inhibitory activity of the test compound was calculated in the same manner as in the HTRF method described above.

### (3) Luciferase Assay (ELISA)

HL-10 cells were treated with the test compound and IL-4 in the same manner as in the chemiluminescence method described above, and 10 µl TBS containing a protease inhibitor cocktail and 2% Triton X-100 was added in place of PicaGene, and the sample was incubated at room temperature for 1 hour. 30 µl of TBS solution (10 µg/ml) containing goat-derived anti-luciferase polyclonal antibody (Chemicon) was added to each well of a 384-well plate (Maxisoap, Nunc) and then left at 4°C overnight. After the antibody solution was removed, each well was blocked with 100 µl TBS containing 0.5% BSA at room temperature for 2 hours. Each well was washed 3 times with 100 µl wash (TBS containing 0.01% Tween 20), and then 30 µl cell sample was added to each well and incubated at 4°C overnight. Each well was washed 3 times with 100 µl wash, and then 30 µl TBS containing 330 ng/ml HRP-labeled goat-derived anti-luciferase polyclonal antibody (Rockland), 0.01% Tween 20 and 0.1% BSA was added to each well which was then incubated at room temperature for 5 hours. After the reaction, each well was washed 3 times with 100 µl wash, and 30 µl TMB substrate solution (Dako) was added to each well and colored at room temperature for 30 minutes. After coloration, the reaction was terminated with 30 µl of 1 N sulfuric acid, and the absorbance at 450 nm was measured with a multi-label counter. The transcriptional inhibitory activity of the test compound was calculated in the same manner as in the HTRF method.

### (4) Measurement of Cell Growth

HL-10 cells were treated with the test compound and IL-4 for 24 hours in the same manner as in the chemiluminescence method, then 3 µl of WST-8 reagent (Kishida Chemical Co., Ltd.) was added to each well, the cells were further cultured for 1 hour, and the absorbance at 450 nm was measured with a multi-label counter. The degree of inhibition of cell growth (%) was calculated assuming that the absorbance obtained from the cells treated with IL-4 without adding the compound was 100%.

Some compounds that were used inhibited cell growth. These compounds were considered to apparently inhibit the transcription of STAT6, and in the HTRF method and in the luciferase assay (ELISA), the compounds inhibiting cell growth also certainly inhibited transcription. However, in the luciferase assay (chemiluminescence method), there were compounds which though inhibiting cell growth, did apparently not inhibit transcription. Among the compounds not inhibiting cell growth, there were compounds which though showing a strong transcriptional inhibitory activity in the HTRF method and in the luciferase assay (ELISA), were not recognized to have a transcriptional inhibitory activity in the luciferase assay (chemiluminescence method), or there are compounds showing a strong inhibitory activity only in the luciferase assay (chemiluminescence method). An estimated reason that the results obtained in the luciferase assay (chemiluminescence method) do not agree with those of the other methods is that the measurement system utilizing chemiluminescence is easily influenced by the test compound.

From the experimental results, it was estimated that the HTRF method of the present invention can be used to perform a screening experiment which is more hardly influenced by a compound and more highly accurate than by the luciferase assay as a general method and enables a screening experiment superior in rapidness and easiness to ELISA.
The results of measurement of the transcriptional inhibitory activities of 15 compounds by the HTRF method and the luciferase methods (chemiluminescence method and ELISA) and the results of measurement of the cell growth inhibitory activities of these compounds are shown in the table below.

**Table 1**

| Compound Name | STAT6 Transcriptional Inhibitory Activity(%) | | | Cell Growth Inhibition (%) |
|---|---|---|---|---|
| | HTRF | Luciferase (Chemiluminescence Method) | Luciferase (ELISA) | |
| A | 99 | < 0 | 87 | 85 |
| B | 98 | < 0 | 99 | 85 |
| C | 98 | < 0 | 41 | 83 |
| D | 94 | 118 | 92 | 75 |
| E | 89 | 105 | 79 | 61 |
| F | 55 | 46 | 60 | 37 |
| G | 64 | < 0 | 46 | 18 |
| H | 74 | 66 | 63 | 15 |
| I | 84 | 81 | 81 | 12 |
| J | 78 | 62 | 44 | 7 |
| K | 62 | 69 | 53 | 3 |
| L | 57 | < 0 | 41 | -12 |
| M | 82 | 19 | 66 | -15 |
| N | 22 | 97 | < 0 | -16 |
| O | 23 | 72 | < 0 | -22 |

SEQ ID NO: 1 is a sequence of a polypeptide used in preparing an antibody.
SEQ ID NO: 2 is a sequence of a polypeptide used in preparing an antibody.
SEQ ID NO: 3 is a sequence of a polypeptide used as an epitope tag.
SEQ ID NO: 4 is an amino acid sequence of a variable region (VH) of 6G4 antibody.
SEQ ID NO:5 is an amino acid sequence of a variable region (VL) of 6G4 antibody.
SEQ ID NO:6 is an amino acid sequence of a variable region (VH) of 2E6 antibody.
SEQ ID NO:7 is an amino acid sequence of a variable region (VL) of 2E6 antibody.
SEQ ID NO: 8 is an oligonucleotide designed to synthesize a cDNA for a VH region of 6G4 antibody.
SEQ ID NO: 9 is an oligonucleotide designed to synthesize a cDNA for a VH region of 2E6 antibody.
SEQ ID NO: 10 is an oligonucleotide designed to synthesize a cDNA for VL regions of 6G4 and 2E6 antibodies.
SEQ ID NO: 11 is a PCR primer designed to amplify a gene for a VH region of 6G4 antibody.
SEQ ID NO: 12 is a PCR primer designed to amplify a gene for a VH region of 2E6 antibody.
SEQ ID NO: 13 is a PCR primer designed to amplify a gene for VL regions of 6G4 and 2E6 antibodies.
SEQ ID NO: 14 is a gene sequence of an epitope tag used in the Examples.
SEQ ID NO: 15 is a gene sequence of a reporter gene used in the Examples.
SEQ ID NO: 16 is a gene sequence of an IL-4 regulatory element of germ line ε promoter for immunoglobulin H chain.
SEQ ID NO: 17 is a gene sequence of a thymidine kinase promoter used in the Examples.

## Claims

1. A reporter gene assay, comprising the steps of:
(a) contacting a cell having a vector wherein a reporter gene containing a gene encoding an epitope tag having a first epitope and a second epitopes is ligated downstream to a recognition sequence of a transcription factor and a nucleotide sequence necessary for transcriptional initiation, with a test substance, a detection antibody recognizing the first epitope and a detection antibody recognizing the second epitope;
(b) detecting a phenomenon caused by both the detection antibodies binding to the first and second epitopes and coming close to each other; and
(c) correlating the detected phenomenon with the effect of the test substance on transcriptional regulatory mechanism,
wherein the first epitope and the second epitope are arranged such that upon binding of their recognizing detection antibodies thereto, both the detection antibodies can come close to each other.

2. The method according to claim 1, wherein the cell is allowed to express a member selected from the group consisting of a transcription factor, a ligand, a receptor and a coactivator.

3. The method according to claim 1 or 2, wherein both the detection antibodies are labeled with a phosphor.

4. The method according to claim 3, wherein the phosphor consists of a combination of a europium compound and an allophycocyanin derivative.

5. A cell comprising a vector wherein a reporter gene containing a gene encoding an epitope tag having first and second epitopes is ligated downstream to a recognition sequence of a transcription factor and a nucleotide sequence necessary for transcriptional initiation.

6. The cell according to claim 5, wherein the reporter gene has a sequence set forth in SEQ ID NO: 15.

7. A vector for use in the method according to any of claims 1 to 4.

8. A kit for use in the method according to any of claims 1 to 4.
